# EUROPEAN PATENT SPECIFICATION

(11) **EP 2 330 986 B1**
(45) Date of publication and mention of the grant of the patent: **13.03.2019**
(21) Application number: 09791831.2
(22) Date of filing: 24.08.2009
(51) Int. Cl.: A61B 17/04, A61B 17/22, A61B 17/29, A61B 17/00

(54) **ENDOSCOPIC SUTURING DEVICE**
ENDOSKOPISCHE NÄHVORRICHTUNG
DISPOSITIF DE SUTURE ENDOSCOPIQUE

(30) Priority: 28.08.2008 US 92557 P; 21.08.2009 US 545310
(43) Date of publication of application: 15.06.2011
(73) Proprietor: Boston Scientific Scimed, Inc., Maple Grove, MN 55311-1566 (US)
(72) Inventor: CLERC, Claude, Marlborough, MA 01752 (US); RIOUX, Robert, F., Ashland, MA 01721 (US)
(74) Representative: Vossius & Partner Patentanwälte Rechtsanwälte mbB
(86) International application number: PCT/US2009/054736
(87) International publication number: WO 2010/025102

(56) References cited:
- EP-A- 1 584 295
- EP-A1- 1 839 590
- WO-A-97/27807
- US-A1- 2002 116 011
- US-A1- 2003 233 104
- US-B1- 6 346 111
- US-B1- 7 048 748

## Description

### Technical Field

The invention generally relates to a medical device, and more particularly to a suturing device for use in endoscopic procedures.

### Background Information

It is known to use suturing devices with rigid shafts in surgical and laparoscopic interventions within the body of a patient. The rigidity of the shafts of such known suturing devices makes insertion and use within certain body lumens difficult or impossible.

There are also known instruments available that allow for viewing certain areas of the human body through, for example, a natural body opening or a small puncture wound, and thus avoid the need for making such large openings. These instruments, called endoscopes, can be used in conjunction with specialized surgical instruments to detect, diagnose, sample, and repair areas of the body that previously required open surgery to access.

Some surgical instruments used in endoscopic procedures are limited by the way they grasp tissue, cut tissue, apply a suture, or recapture the needle and suture. Furthermore, many surgical instruments are complicated and expensive to use due to the numerous parts and/or subassemblies required to make them function properly. Suturing, in particular, remains a delicate and time-consuming aspect of most surgeries, including those performed endoscopically.

In addition, many surgical and suturing instruments are limited by the manner in which they access the areas of the human body in need of diagnosis, sampling, treatment, or repair. In particular, the instruments may not be able to access tissue or organs that are located deep within the body or that are in some way obstructed. Such instruments typically have a rigid shaft, and do not allow for efficient orientation to reach the location of diagnosis, sampling, treatment, or repair.

From US 2003/233104 A1 a suturing device is known including a distal portion that is deflectably and/or pivotably coupled to the remainder of the instrument for improved manoeuvrability and functionality during surgery. From US 7048748 B1 a surgical instrument is known that automatically ties interrupted sutures. Sutures, a needle and two small blades come in a modular unit that is attached to a main piece. EP 1 584 295 A2 discloses a ligature and suture device including a distal end insertion portion, a needle body, a ligature tool and an operation section. The operation section includes a housing, a puncture handle that is provided in the housing, a pressing handle that is provided in the housing, a ligature tool operation unit that is provided in the housing, and a ligature handle that is provided in the ligatures tool operation unit.

### Summary of the Invention

The invention relates to a suturing device according to claim 1.

In one aspect, the invention involves a suturing device. The suturing device includes a head having a proximal end portion and a distal end portion. The proximal end portion defines a first opening and the distal end portion defines a second opening. The proximal end portion of the head is configured to be removably coupled to a distal end portion of an elongate medical device configured to be disposed within a body lumen of a patient. The suturing device also includes a curved carrier member movably disposed at least partially within the distal end portion of the head. The carrier member is movable from a first position in which the carrier member is disposed substantially within the distal end portion of the head to a second position in which a first portion of the carrier member is moved out through the second opening and disposed adjacent the first opening, and a second portion of the carrier member is disposed within the distal end portion of the head. The carrier member is configured to receive a needle coupled to a suture when in the first position and configured to pass the needle and a portion of the suture through a tissue when moved from the first position to the second position.

The suturing device further includes a coupling member configured to removably couple the head to the medical device. In some embodiments, the carrier member can define an opening configured to receive a needle coupled to a suture. The suturing device further includes an elongate flexible sheath coupled to the head and an actuation cable coupled to the carrier member. The actuation cable can be disposed at least partially within a lumen defined by the elongate flexible sheath. The actuation cable can be configured to extend through a lumen of the medical device when the head is removably coupled to the medical device. The carrier member can be a first carrier member and the suturing device can include a second carrier member removably disposed at least partially within the distal end portion of the head. The carrier member, when moved between its first position and its second position, can rotate about an axis orthogonal to a longitudinal axis defined by the head. The head can include a first portion and a second portion and the first portion of the head is configured to move relative to the second portion. The suturing device can further include an imager coupled to the head. The suturing device can further include the medical device and the head can be removably coupled to the medical device.

Also disclosed is a suturing device including a flexible elongate sheath having a proximal end portion, a distal end portion, and defining a lumen therethrough. The suturing device includes a head configured to be coupled to the distal end portion of the flexible elongate sheath. The head is configured to be disposed within a body lumen of a patient. The suturing device includes a carrier member movably coupled to the head. The carrier member is configured to pass a suture through a tissue within the body lumen. The suturing device also includes an expandable member coupled to an exterior surface of the head. The expandable member has a collapsed configuration and an expanded configuration.

Examples according to this aspect of the disclosure can include the following features. The expandable member can be configured to secure the head within a body lumen when in its expanded configuration. The suturing device can further include an actuation cable at least partially disposed within the lumen of the flexible elongate sheath. The actuation cable can be configured to move the carrier member between a first position and a second position and is configured to pass the suture through the tissue when moved from its first position to its second position. The suturing device can further include an imager coupled to the head and configured to capture an image of the body lumen when the head is disposed within the body. The flexible elongate sheath can define a lumen configured to receive a guide wire therethrough. The carrier member can be a first carrier member and the suturing device can further include a second carrier member coupled to the head. The second carrier member can be configured to pass a suture through a tissue within the body lumen. The head of the suturing device can also be configured to be removably coupled to an endoscope.

Also disclosed is a suturing device including an elongate body configured to be inserted into a body lumen of a patient. The elongate body has a flexible portion and a substantially rigid portion disposed at a distal end portion of the elongate body. The elongate body includes a retaining portion configured to receive a guide wire for guiding the elongate body within the body lumen. The suturing device also includes a carrier member movably disposed at least partially within the substantially rigid portion of the elongate body. The carrier member is configured to pass a needle through a tissue within the body lumen.

Examples according to this aspect of the disclosure can include the following features. The retaining portion can include a lumen defined collectively by the flexible portion of the elongate body and the substantially rigid portion of the elongate body. In such an example the suturing device can further include an opening defined by the substantially rigid portion of the elongate body. The opening can be in communication with the lumen. The lumen can be configured to receive the guide wire therethrough such that a distal end portion of the guide wire extends outside the opening of the substantially rigid portion of the elongate body. In some examples, the retaining portion can be disposed on an exterior surface of the substantially rigid portion of the elongate body and can be configured to slidably receive the guide wire therethrough. The suturing device can further include an actuation cable coupled to the carrier member. The actuation cable can be configured to move the carrier member between a first position and a second position. The carrier member can be configured to pass the suture through the tissue when moved from its first position to its second position. The actuation cable can be at least partially disposed within a lumen defined by the flexible portion of the elongate body. The suturing device can further include an imager coupled to the substantially rigid portion of the elongate body. The suturing device can further include an expandable member coupled to an exterior surface of the substantially rigid portion of the elongate body. The expandable member can have a collapsed configuration and an expanded configuration. The expandable member in the expanded configuration is configured to secure a position of the substantially rigid portion of the elongate body within a body lumen. The elongate body of the suturing device can be configured to be removably coupled to an endoscope or other elongate medical device.

For a fuller understanding of the nature and operation of the invention and also various possible embodiments according to the invention, reference is made to the drawings briefly described in the next section and also to the more detailed description that follows the brief description of the drawings.

### Brief Description of the Drawings

In the drawings, the same or similar reference numbers generally denote the same or similar elements of the various disclosed embodiments. The drawings are not necessarily to scale, emphasis instead generally being placed on conveying certain concepts and aspects according to the invention.
FIG. 1 is a schematic illustration of a suturing device according to an embodiment of the invention.
FIG. 2 is a side view of a portion of a suturing device according to an embodiment of the invention shown coupled to another medical device.
FIG. 3 is a cross-sectional view of the distal end portion of the suturing device of FIG. 2 and the distal end portion of the other medical device .
FIG. 4 is a cross-sectional view of the proximal end portion of the suturing device of FIG. 2.
FIG. 5 is a side view of a needle coupled to a portion of a suture for use in a suturing device in accordance with the invention.
FIG. 6 is a side perspective view of a catch member of a suturing device and the needle and portion of a suture of FIG. 5.
FIG. 7 is a side cross-sectional view of a distal end portion of the suturing device of FIG. 2 showing a carrier member and needle in a first position.
FIG. 8 is a side cross-sectional view of the distal end portion of the suturing device of FIG. 8 showing the carrier member and needle in a second position.
FIG. 9A is a side view of a suturing device shown coupled to an endoscope device.
FIG. 9B is a side-cross-sectional view of a distal end portion of the suturing device and endoscope device of FIG. 9A.
FIG. 10 is a side perspective view of a portion of a suturing device.
FIG. 11 is a cross-sectional view of the portion of the suturing device of FIG. 10.
FIG. 12 is a perspective view of a distal end portion of the suturing device of FIG. 10.
FIG. 13 is a side perspective view of a portion of a suturing device.
FIG. 14 is a side perspective view of a portion of a suturing device including an expandable member illustrating the expandable member in an expanded configuration.
FIG. 15 is a side perspective view of the portion of the suturing device of FIG. 14 illustrating the expandable member in a collapsed configuration.
FIG. 16 is a side perspective view of a portion of a suturing device including a lens assembly.
FIG. 17 is a cross-sectional view of a distal end portion of the suturing device of FIG. 16.
FIG. 18 is a side-perspective view of a portion of a suturing device.
FIG. 19 is a side view of a portion of a suturing device.
FIG. 20 illustrates a suturing device shown in a collapsed configuration and inserted at least partially within a schematic illustration of an esophagus.
FIG. 21 illustrates the suturing device of FIG. 20 shown in an expanded configuration within a schematic illustration of an esophagus and being used to secure an esophageal stent.
FIG. 22 is an end view of an embodiment of a coupling member.
FIG. 23 is a side view of another embodiment of a coupling member.

### Description

The invention generally relates to a suturing device that can be used for suturing a tissue within a patient's body and/or for securing a medical device (such as a stent or implant) within a patient's body with sutures. For example, a suturing device can be used to place sutures within a body lumen. A suturing device can also be used to secure an implant to a wall of a body lumen, such as an esophagus. Various configurations of a suturing device are described herein that can be used to place a suture within a patient's body such as in a lumen, passage, cavity or other area within a patient's body. The patient can be a mammal, and typically is a human.

A suturing device according to the invention is configured to be removably coupled to an elongate medical device, such as an endoscope, that can be inserted into a body lumen of a patient, such as, for example, an esophagus. Such an elongate medical device can be flexible or rigid. The suturing device includes a coupling member configured to removably couple the suturing device to the endoscope. In other embodiments, the suturing device includes a head and an elongate flexible sheath coupled thereto. The suturing device can be configured to receive a guide wire to be used to guide and maneuver the suturing device to a desired treatment site within a body.

In some examples, a suturing device can also be configured to provide imaging capabilities to enable visual inspection and magnification of a cavity in the body of the patient. In some examples, a suturing device includes an expandable member coupled to a head at a distal end portion of the suturing device. The expandable member can be used to maintain the position of the head at a desired location within the patient's body. The expandable member can be, for example, an inflatable balloon.

As used herein, the words "proximal" and "distal" refer to direction closer to and away from, respectively, an operator (e.g., surgeon, physician, nurse, technician, etc.) who would insert the disclosed suturing device into the patient, with the distal end of the device inserted first into a patient's body. The end of the suturing device inserted first inside a patient's body would be the distal end of the device, and the end of the device closest to the operator and to an exterior incision or opening in the patient's body would be the proximal end of the suturing device.

In the embodiments, a suturing device includes a head configured to be removably coupled to an elongate medical device, such as an endoscope. The suturing device includes a coupling member configured to removably couple the head to the elongate medical device. The suturing device also includes an elongate flexible sheath coupled to the head. The elongate flexible sheath can extend along an exterior of the medical device (to which the suturing device is coupled), or can be disposed within a lumen defined by and extending through the medical device, in other examples.

In the embodiments, the suturing device includes a head and an elongate flexible sheath coupled thereto. The suturing device further includes a retaining portion configured to receive a guide wire or otherwise couple a guide wire to the head. A guide wire can extend along the exterior of the head and/or elongate flexible sheath of the suturing device, or can be disposed within a lumen defined by the head and/or a lumen defined by the sheath.

The various devices described herein can be used, for example, as an endoscopic suturing device for suturing tissue or securing implants or stents, such as esophageal stents, but are not limited to such use. For example, the devices and methods can be used for securing Tannenbaum stents, duodenal stents, biliary stents, or colonic stents. The devices and methods can be used with other types of implants as well.

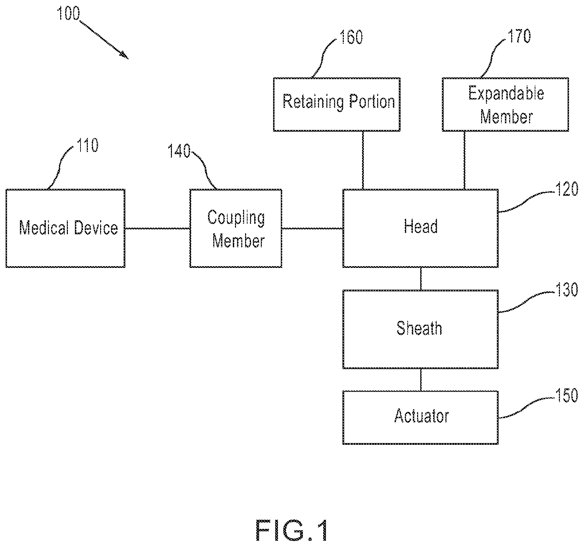

FIG. 1 is a schematic illustration of various components that can be used in a suturing device for endoscopic suturing. A suturing device according to the invention can have a variety of different configurations and include a variety of different components. One suturing device 100 according to the invention can include a head 120, a flexible elongate sheath 130, and an actuator 150. The head can have a variety of different shapes, sizes, and configurations. The head 120 can be constructed with one or more components and can be substantially rigid. The head 120 can define an interior region in which components of the suturing device 100 are operatively disposed. In some embodiments, the head 120 includes a curved or substantially C-shaped portion. In some embodiments, the head 120 includes a first portion and a second portion that is configured to articulate or rotate relative to the first portion. In some embodiments, the head 120 is configured to articulate relative to the flexible elongate sheath 130.

The actuator 150 can include an actuation cable (not shown in FIG. 1) disposed at least partially within a lumen of the elongate flexible sheath 130 and coupled at a distal end portion to the head 120. The actuator 150 can also include a handle and pusher member (not shown in FIG. 1) that can be used by a medical practitioner to actuate a suturing mechanism (not shown in FIG. 1) coupled to the head 120. The suturing mechanism can include one or more carrier members coupled to and disposed within an interior region of the head 120, and a catch member (not shown in FIG. 1) coupled to the head 120. The suturing mechanism can be used to pass a needle and a suture through a tissue. For example, a needle (e.g., a trocar or arrow needle) that is coupled to a length of suture can be releasably coupled to the carrier member within the head 120, and the carrier member can be actuated to move the needle through a portion of tissue. Other types of fasteners and/or needles can alternatively be coupled to a suture and placed within a tissue using a suturing device 100. For example, a T-fastener can be coupled to a length of suture.

The actuation of the carrier member can, for example, include a spring-loaded actuation mechanism, a hydraulic actuation mechanism, pneumatic actuation mechanism, or an electromechanical actuation mechanism. The various components and operation of the suturing mechanism are described in more detail below with reference to specific embodiments.

The suturing device 100 can be removably coupled to an elongate medical device 110 with a coupling member 140. The coupling member 140 can be, for example, a band, a clamp, a clip, or any other suitable connector or coupling device. For example, in some embodiments, the suturing device 100 can be coupled to an elongate medical device 110 with screws or other types of threaded attachment methods, an interference fit, compressed tabs, adhesives, magnets, hose clamps, etc. In some embodiments, more than one coupling member 140 can be used to removably couple the suturing device 100 to another medical device (e.g., medical device 110). In some embodiments, the coupling member 140 is elongate and can extend along a length or a portion of a length of the suturing device 100 and/or the medical device to which the suturing device 100 is to be coupled. For example, such an elongate coupling member can include two clamp portions; one to receive a portion of the suturing device, the other to receive a portion of the medical device to which the suturing device 100 is to be removably attached.

In some embodiments, the coupling member 140 can be formed integrally or monolithically with the head 120. For example, the head 120 can include an integrally formed clamp portion to removably couple the suturing device 100 to the elongate medical device 110. In alternative examples, the head 120 can be fixedly coupled to an elongate medical device and provided to a user as a single device. The elongate medical device 110 can be flexible or rigid and can be configured to be inserted into a body lumen of a patient such that when the head 120 is coupled to the elongate medical device 110 it can be used to direct or maneuver the head 120 to a desired location within a body lumen. In some embodiments, the elongate medical device 110 is a flexible endoscope. When the head 120 is coupled to a medical device 110, the flexible elongate sheath 130 can be configured to extend through a lumen of the medical device 110 or can extend along an exterior of the medical device 110. In some embodiments, the flexible elongate sheath 130 can be formed with an elastic material such that it can be bent into a desired shape prior to insertion into the patient's body.

In some examples a suturing device 100 can be inserted into a body lumen independently from another medical device such as medical device 110. In such an example the suturing device 100 can include a retaining portion 160 that can be used to releasably couple a guide wire (not shown in FIG. 1) to the suturing device 120. For example, the retaining portion 160 can be a lumen defined collectively by the head 120 and at least a portion of the flexible sheath 130. In some examples, the retaining portion includes a lumen defined only by the head 120. For example, the head 120 can define a guide wire lumen in communication with an opening on a distal end portion of the head 120 and an opening on a proximal end portion of the head 120, and a guide wire can extend therethrough. In some embodiments, the retaining portion 160 includes a retaining member (not shown in FIG. 1) disposed on an exterior of the head 120. For example, one or more connectors that define a channel or lumen can be disposed on an exterior of the head 120 and/or flexible elongate sheath 130 that can receive a guide wire therethrough.

In some examples, the suturing device 100 includes an expandable member 170. The expandable member 170 can be coupled to, for example, an exterior portion of the head 120. The expandable member 170 has a collapsed configuration for insertion into and maneuvering within a patient's body, and an expanded configuration for maintaining a position of the head 120 at a desired treatment site. For example, the expandable member 170 can be expanded within a body lumen such that it contacts a wall of the body lumen and secures the head 120 at a desired location for suturing at the treatment site. The expandable member 170 can be, for example, an inflatable balloon, that can be inflated, for example, with a liquid, gas, gel, etc. The expandable member 170 can alternatively be a mechanically actuated expandable device. For example, the expandable member 170 can include expandable wire arms that can be actuated to assume an expanded configuration that has a greater outer diameter or outer profile than when the expandable device is in a collapsed configuration.

In some embodiments, the suturing device 100 also includes an imaging device (not shown in FIG. 1) coupled to or in the proximity of the head 120. The imaging device can include an image sensor coupled to the head 120 and used to send image signals associated with an image of the interior of the patient's body. The suturing device 100 can also optionally include an ultrasound sensor (not shown in FIG. 1) that can be used to monitor and ensure that a needle (loaded onto the suturing device) does not get pushed through, for example, an aorta or other unintended body structure. Other types of imaging devices can also be included, such as, for example, imaging devices using light, sound, radiation heat or electromagnetic energy can be included.

The flexibility of the flexible elongate sheath 130 allows the suturing device 100 to be inserted into, for example, a body lumen of a patient, or other locations that may not be easily accessible. The flexibility of the elongate flexible sheath 130 also allows the suturing device 100 to be used in conjunction with a flexible medical device, such as an endoscope. Such an embodiment, enables a medical practitioner to perform various endoscopic procedures (e.g., imaging, irrigating, etc.), as well as suturing procedures with a single instrument (e.g., the combined endoscope and suturing device) inserted into the patient's body.

Having described above various general examples, several examples of specific embodiments are now described. These embodiments are only examples, and many other configurations of a suturing device are contemplated.

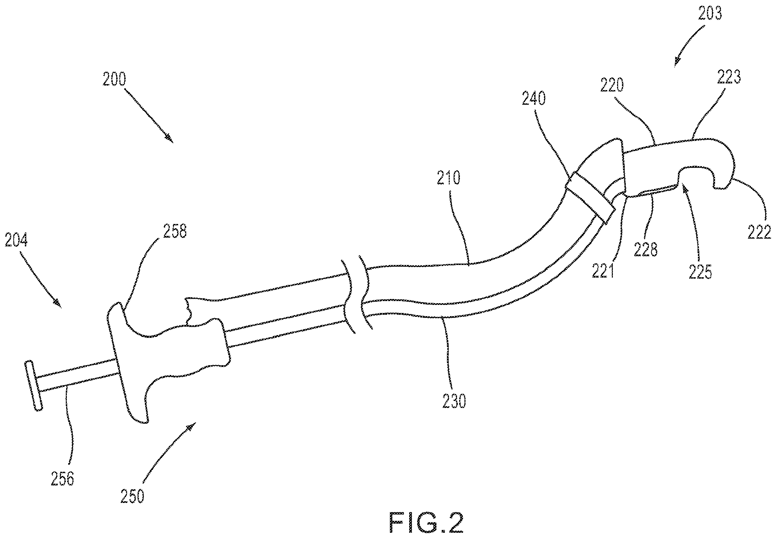

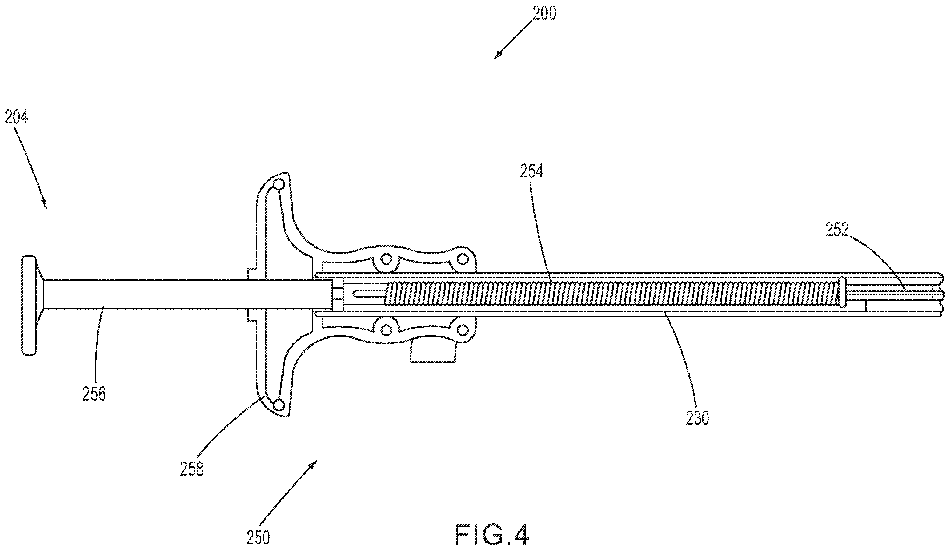

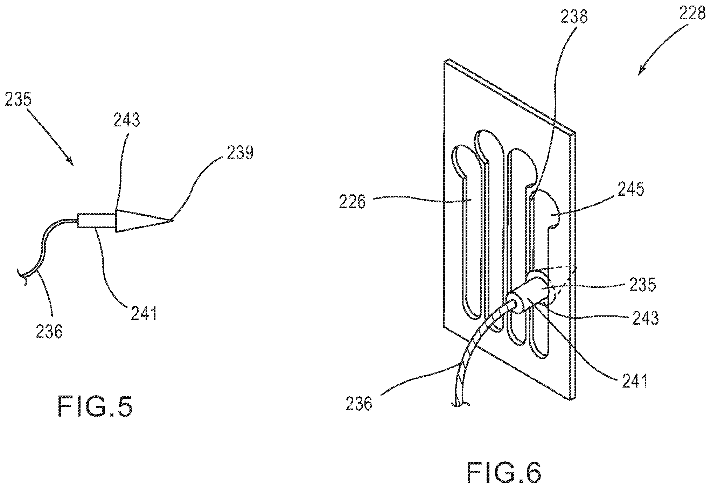

FIGS. 2-8 illustrate a suturing device according to an embodiment of the invention. As shown in FIG. 2, a suturing device 200 includes a distal end portion 203 and a proximal end portion 204. The suturing device 200 includes a head 220, a flexible elongate sheath 230, and an actuator 250. The head 220 is disposed at the distal end portion 203 and is coupled to the flexible elongate sheath 230 and the actuator 250. The actuator 250 includes a spring loaded actuation cable 252 (shown in FIGS. 4 and 5) that extends from the proximal end portion 204 to the distal end portion 203 of the suturing device 200, and a spring 254 (shown in FIG. 4) that is coupled to a proximal end portion of the actuation cable 252.

The actuator 250 also includes a handle 258 disposed at the proximal end portion 204, and a pusher member 256 that is coupled to the handle 258 and is slidably movable within the handle 258. The handle 258 can be a variety of different configurations, for example, the handle 258 can be any one of the types used with Boston Scientific Corporation suturing systems. The pusher member 256 is configured to be pushed or moved distally and to extend longitudinally through at least a portion of the handle 258 and within a portion of the elongate flexible sheath 230. The pusher member 256 is further configured to move the actuation cable 252 distally as described below. In alternative embodiments, other portions of the actuator 250 can be disposed within the head 220 and in some embodiments, one or more portions of the actuator 250 can be attached to or within the elongate medical device 210.

In this embodiment, the suturing device 200 is configured to be releasably coupled to an elongate medical device 210 with a coupling member 240. The elongate medical device 210 can be for example, a flexible elongate endoscope. The coupling member 240 in this embodiment is a band that surrounds the flexible elongate sheath 230 and the elongate medical device 210. The band can be, for example, elastic, such that it can secure the suturing device 200 the elongate medical device 210 with an elastic force. The band can alternatively or in addition, include a first end and a second end, and a fastener to releasably couple the first end to the second end. For example, the fastener can include mating portions of a VELCRO attachment, or mating snap connections, etc. As stated above, other methods of coupling the suturing device 200 to another medical device can alternatively be used, such as a separate clamp member or a clamp formed integrally or monolithically with the head 220. More than one coupling member 240 can also be used.

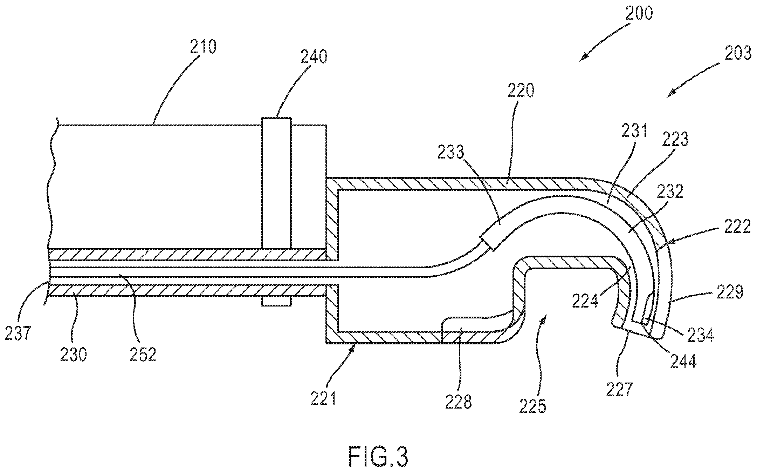

As shown in FIGS. 2 and 3, the head 220 is disposed at a distal end of the suturing device 200 and includes a distal end portion 222 and a proximal end portion 221. The head 220 is substantially rigid and defines an interior region. The distal end portion 222 of the head 220 includes a curved portion 223 that defines a needle exit port or opening 227 and a slot 229, as shown in FIG. 3. The curved portion 223 also defines an interior channel 224 in communication with both the slot 229 and the exit port 227 as best shown in FIGS. 3, 7 and 8. The proximal end portion 221 of the head 220 includes a catch member 228 that includes ribs 238 that define multiple openings 226 (shown in FIG. 6). The distal end portion 222 and the proximal end portion 221 of the head 220 collectively define an opening 225 for receiving tissue, as described in more detail below.

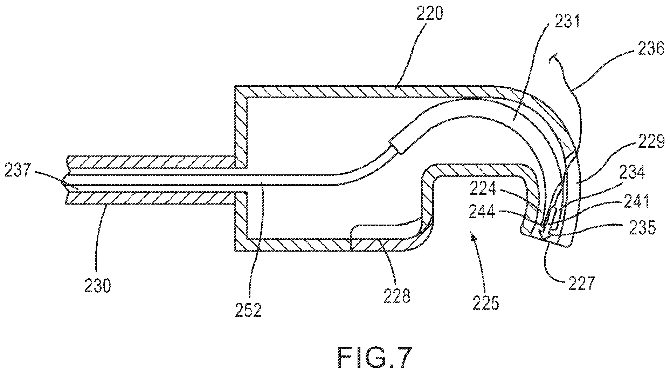

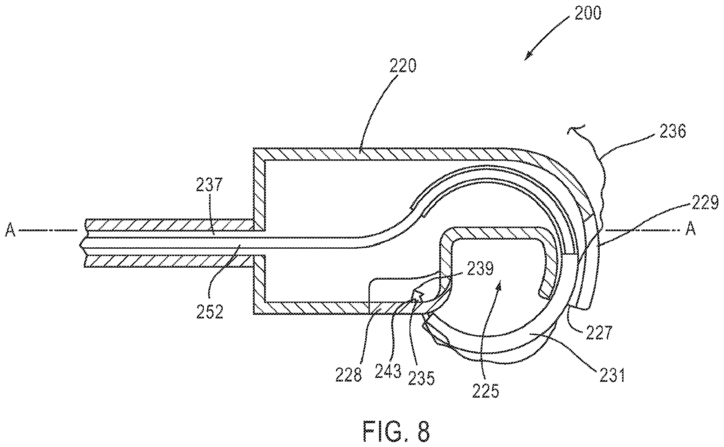

As shown in FIG. 3, a curved needle carrier member 231 is disposed within the head 220 and is coupled to a distal end of the actuation cable 252. The carrier member 231 has a distal portion 232 and a proximal portion 233, and is movable within the channel 224 of the curved portion 223. The proximal portion 233 of the carrier member 231 is coupled to the actuation cable 252, and the distal portion 232 is configured to be moved or extended out of the needle exit port 227 when the actuator 250 is actuated (described in more detail below). The distal portion 232 of the carrier member 231 defines an opening or slot 234 and an opening 244 at a distal end in communication with the slot 234 configured to receive a needle 235 (shown in FIGS. 5-8). The opening 234 of the carrier member 231, in some embodiments can maintain the needle 235 within the opening 244 by, for example, a slight friction fit, although any other suitable coupler or connecter can be used. The slot 229 is configured to allow a suture 236 (shown in FIGS. 6-9) coupled to the needle 235 to extend out of the curved portion 223 and through the slot 229 of the head 220 (shown in FIGS. 7 and 8).

The flexible elongate sheath 230, as shown in FIG. 3, defines a lumen 237 extending between a proximal end and a distal end of the sheath 230. The actuation cable 252 extends longitudinally through the lumen 237 of the sheath 230 and into the proximal end portion 221 of the head 220. The proximal end portion of the actuation cable 252, as stated above, is coupled to the spring 254 as illustrated in FIG. 4. The actuation cable 252 is also coupled to the proximal portion 233 of the carrier member 231 to extend the distal end portion 232 of the carrier member 231 out of the needle exit port 227 upon actuation. The actuation cable 252 enables the carrier member 231 to move between a first position (i.e., a retracted position within the distal end portion 222 of the head 220) (shown in FIG. 7) and a second position (i.e., a deployed position in which the distal end of the carrier member 231 is disposed adjacent the catch member 228). During movement between the first position and the second position, the carrier member 231 rotates about an axis transverse (e.g., orthogonal) to a longitudinal axis A-A defined by the head 220 (shown in FIG. 8).

FIG. 5 illustrates an example embodiment of a needle 235 that can be loaded onto the suturing device 200 and passed through a portion of tissue within a patient. The needle 235 includes a tip 239 and a shaft 241 and is coupled to a suture 236. The needle 235 is inserted through the slot 229 of the head and at least partially within opening 234 of the carrier member 231 and partially within the channel 224 defined by the head 220 (as shown in FIG. 8). The needle 235 can be held by a slight friction fit. The suture 236 extends out of the slot 229 of the head 220 as shown in FIGS. 7 and 8.

With the needle 235 and suture 236 loaded on the suturing device 200 as shown in FIG. 8, and with the suturing device 200 coupled to an elongate medical device, such as medical device 210 (not shown in FIG. 7), a distal end portion of the suturing device 200 and medical device 210 can be inserted into a patient's body. The user positions the distal portion of the suturing device 200 at a desired tissue site such that a portion of tissue is disposed within the opening 225 defined by the head 220, and then actuates the actuator 250. Actuation is accomplished by pushing on the pusher 256 thereby moving the actuation cable 252 in a distal direction. The actuation cable 252 then slidably moves the distal end portion 232 of the carrier member 231 (and the needle 235) through the needle exit port 227 and through the portion of the tissue within the opening 225. The user continues to push the pusher 256 until the needle 235 enters the catch member 228 through the openings 226 of the catch member 228 as shown in FIGS. 6 and 8 (FIG. 6 shows the carrier member 228 substantially planar for illustrative purposes). The ribs 238 deflect slightly to allow the needle 235 to pass through. After the shoulder 243 of the needle 235 has passed the ribs 238, the ribs 238 spring back to their original position defining the openings 226, and the needle 235 remains captured in the catch member 228. Although the catch member 228 is shown as including ribs 238, the catch member 228 (and other catch members described herein) can alternatively include, for example, a puncturable membrane, a screen, or openings having a variety of different shapes and/or sizes, that can be engaged by and capture the needle 235.

Still describing the operation of the suturing device 200 with respect to FIGS. 5-8, as the pusher 256 is released or pulled in a proximal direction, the actuation cable 252 is moved proximally. The carrier member 231 is then moved proximally to the retracted position described above. As the carrier member 231 returns to its first position (i.e., retracted position), the needle 235 slides out of the opening 234 of the carrier member 231 and remains held within the opening 226 of the catch member 228.

To remove the needle 235 from the catch member 228, the needle 235 may be removed via an enlarged portion 245 of the opening 226 (shown in FIG. 6). The enlarged portion 245 is sized to allow the formed shoulder 243 to pass through without resistance.

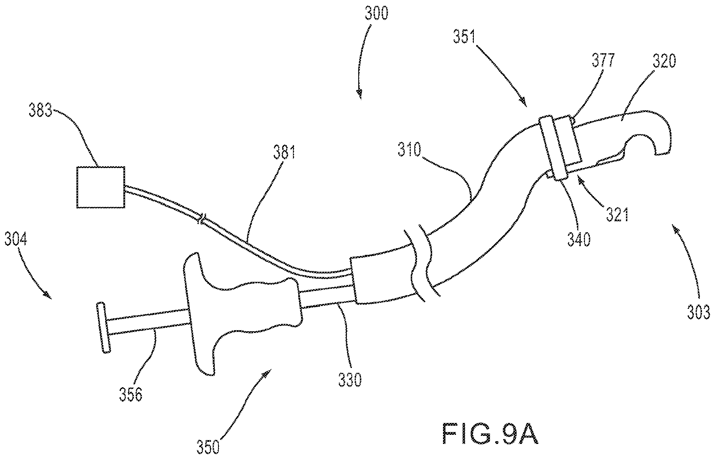

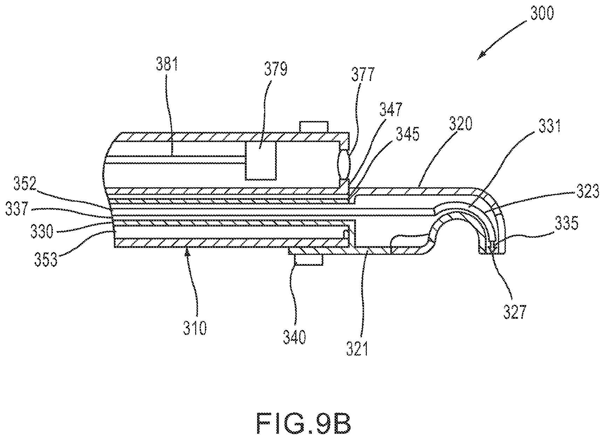

FIGS. 9A and 9B illustrate another example of a suturing device. In this example a suturing device 300 is shown removably coupled to a flexible endoscope 310. The suturing device 300 includes a head 320 disposed at a distal end portion 303, an elongate flexible sheath 330 and an actuator 350. The actuator 350 includes a spring-loaded actuation cable 352 (shown in FIG. 9B), and a handle 358 and a pusher member 356 disposed at a distal end portion 304 of the suturing device 300. The head 320 and actuator 350 are structurally similar to the head 220 and actuator 250 previously discussed, and thus will not be discussed in detail below.

The endoscope 310 includes a lens assembly 377 and an imager 379 (shown in FIG. 9B). As shown in FIG. 9A, a proximal end portion 351 of the endoscope 310 can be coupled to a control device 383 (shown schematically) via a cable 381. The control device 383 can include, for example, controls for operating the imager 379 and a processor for processing image signals produced by the imager 379. The control device can also include a monitor or be coupled to a monitor (not shown) for viewing the processed images. It should be understood that the endoscope 310 is merely an example of the type of endoscope to which the suturing device 300 can be coupled. For example, an endoscope having an imager such as, for example, a charge coupled device (CCD), a complementary metal-oxide-semiconductor (CMOS) sensor, an active pixel sensor, a thermal imaging sensor, a video camera tube, a gamma camera sensor, an x-ray sensor can be used. In some embodiments, an endoscope using optical fibers can be used. It should also be understood that other types of medical devices can alternatively be coupled to the suturing device 300 depending on the particular procedure to be performed.

In this example, the endoscope 310 also defines a lumen 353 between its proximal end portion 351 and distal end portion 347, and an opening 345 at its distal end. The distal end portion 347 is configured to be coupled to a proximal end portion 321 of the head 320. The opening 345 and lumen 353 of the endoscope 310 are configured to removably receive the elongate flexible sheath 330 therethrough when the suturing device 300 is coupled to the endoscope 310. As with the previous example, the elongate flexible sheath 330 defines a lumen 337 therethrough. The actuation cable 352 is disposed within the lumen 337 and is coupled to a carrier member 331. As described above, when the suturing device 300 is actuated, the actuation cable 352 moves the carrier member 331 (shown with a needle 335 coupled thereto) out of a needle exit port 327 defined at a distal end portion 323 of the head 320.

The suturing device 300 and endoscope 310 can be used in a similar manner as described above for previous embodiments. For example, a distal end of the suturing device 300 and the endoscope 310 combination can be inserted into a body lumen. The endoscope 310 can be used to capture images of the body lumen and/or help locate a desired treatment site within the body lumen. The suturing device 300 can then be actuated as previously described to place sutures at the treatment site (e.g., suture tissue and/or secure an implant or stent (or other device) within the body lumen).

In an alternative example the suturing device 300 may not include a flexible elongate sheath 330. Instead, the actuation cable 352 can extend through only the lumen 353 of the endoscope 310. Also, although the suturing device 300 is shown coupled to an endoscope, it should be understood that suturing device 300 can alternatively be coupled to other types of elongate medical devices.

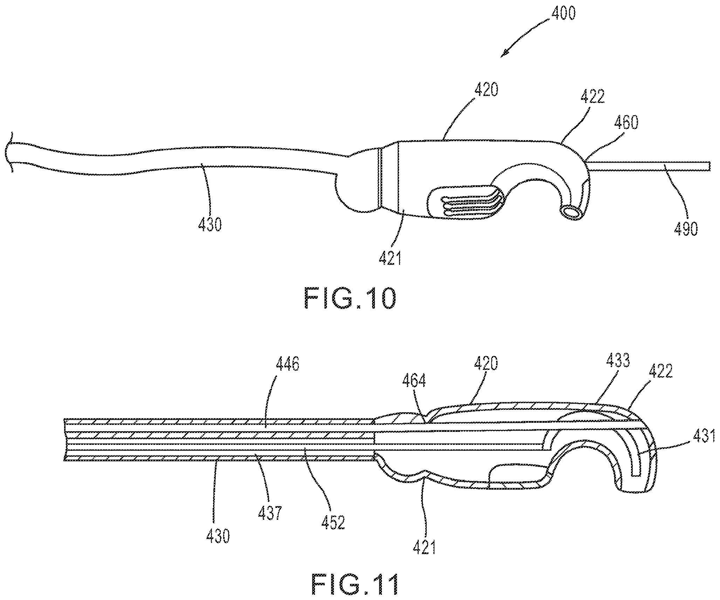

In some examples, as shown in FIGS. 10-17, a suturing device is configured to be inserted into a body of a patient independently of an elongate medical device (e.g., such as medical devices 210 or 310 described above). In such an example a guide wire can be used to assist in guiding a suturing device within a patient's body (e.g., through a lumen of the patient's body). For example, a guide wire can be inserted into a desired location within a patient's body and the suturing device can be slidably coupled to the guide wire and moved along the guide wire to the treatment site. The suturing device can then be actuated to place a suture at the tissue site. The suturing devices illustrated in FIGS 10-17 each include a head, a suturing mechanism (e.g., a carrier member and a catch member), an elongate flexible sheath and an actuator, that are functionally and structurally similar to the embodiments previously described. Thus, the structure and function of these components are not described in detail with reference to these embodiments.

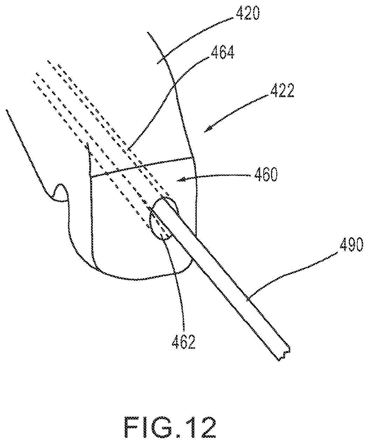

Referring to FIGS. 10-12, a suturing device 400 includes a head 420 and a retaining portion 460 configured to retain a guide wire 490. In this example, the retaining portion 460 includes an opening 462 (shown in FIGS. 11 and 12) defined by a distal end portion 422 of the head 420. The opening 462 can slidably receive the guide wire 490 (not shown in FIG. 11) therethrough. The retaining portion 460 further includes a lumen 464 (shown in FIG. 12) defined by and extending through the head 420 and in communication with the opening 462, and a lumen 446 defined by and extending through the elongate flexible sheath 430. The lumen 446 is in communication with the lumen 464 and thus collectively define a continuous lumen. The elongate flexible sheath 430 also defines an opening (not shown) on a proximal end portion of the elongate flexible sheath 430 that is in communication with the lumen 446. The guide wire 490 can be slidably received through the opening 462, the lumen 464, the lumen 446 and the opening on the proximal end portion of the elongate flexible sheath 430.

As with the previous embodiments, as shown in FIG. 11, the sheath 430 also defines a lumen 437 configured to retain an actuation cable 452 The actuation cable 452 extends longitudinally through the elongate flexible sheath 430 and into the proximal end portion 421 of the head 420. The actuation cable 452 is configured to be coupled to a proximal end portion 433 of the carrier member 431 disposed within the head 420.

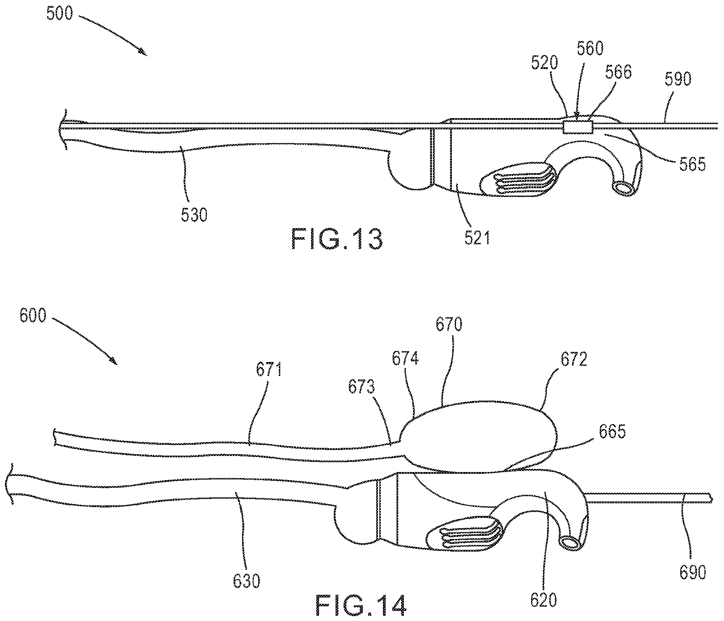

Referring to FIG. 13, a suturing device 500 includes a head 520 and a retaining portion 560 configured to retain a guide wire 590. An elongate flexible sheath 530 is coupled to a proximal end portion 521 of the head 520. In this example, the retaining portion 560 includes a retaining member 566 disposed on an exterior surface 565 of the head 520. The retaining member 566 defines a channel or lumen therethrough (not shown). The lumen of the retaining member 566 is configured to slidably receive the guide wire 590 therethrough. As illustrated in FIG. 13, the guide wire 590 extends along an exterior surface of the elongate flexible sheath 530. Although only one retaining member 566 is shown in FIG. 13, a suturing device can include more than one retaining member along an exterior of the suturing device. For example, additional retaining members 566 can be disposed along an exterior surface of the head 520 and/or along the elongate flexible sheath 530.

Although not illustrated in FIGS. 10-13, the suturing devices 400 and 500 can be configured to be removably coupled to an elongate medical device, such as an endoscope, as described above for suturing devices 100, 200 and 300. For example, the suturing devices 400 and 500 can include a coupling member (e.g., 140, 240) as described above for previous embodiments that can be used to couple the suturing device to an elongate medical device.

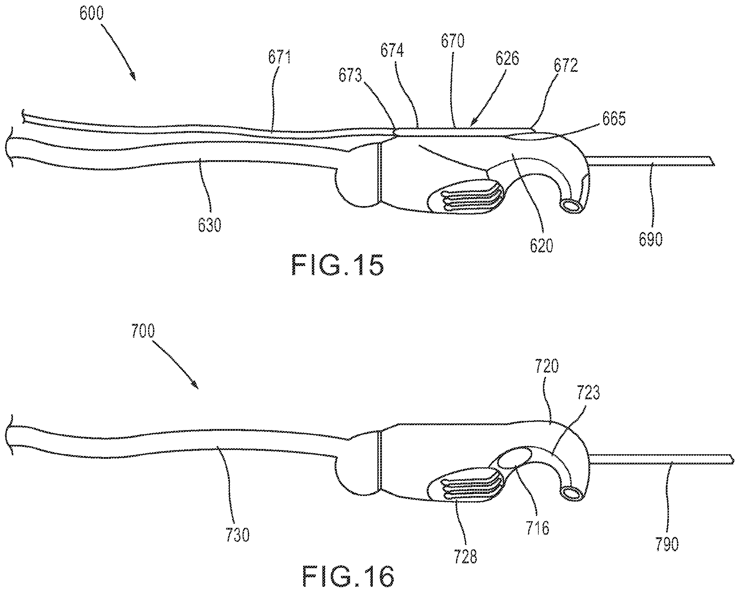

In some examples, a suturing device can include an expandable member that can be used to maintain a position of the suturing device within a body lumen of a patient. As illustrated in FIGS. 14 and 15, a suturing device 600 includes a head 620, an elongate flexible sheath 630 and an expandable member 670 coupled to an exterior surface 665 of the head 620. In this embodiment, the suturing device 600 also includes a retaining portion (not shown) similar to the retaining portion 460 described above for receiving a guide wire 690 therethrough.

The expandable member can be, for example, an inflatable balloon. The expandable member 670 can be coupled to the head 620 with, for example, an adhesive or an elastic strap or any other suitable coupling method. The expandable member 670 can be used to ensure that the head 620 maintains its position within a body lumen. Although the expandable member 670 is described as an inflatable expandable member, other configurations of an expandable member can alternatively be incorporated. For example, the expandable member can be a mechanically actuated device. In some embodiments, the expandable member includes an expandable cage or basket. In some examples, the expandable member is not coupled to the suturing device 600, but rather is provided as a separate component. For example, an expandable member can be extended through a lumen or coupled to a medical device to which the suturing device 600 is coupled. In another example, the expandable member can be separately extended through a body lumen along side the suturing device 600.

The expandable member 670 includes a distal end portion 672 and a proximal end portion 674. A flexible tubular member 671 extends from the proximal end portion 674 of the expandable member 670. The flexible tubular member 671 can be a separate component or monolithically formed with the expandable member 670. The flexible tubular member 671 includes a distal end 673 and a proximal end (not shown) that is configured to extend outside of the patient's body. The tubular member 671 is configured to communicate an inflation medium (e.g., liquid, gas, or gel) to and from the expandable member 670 for expanding or collapsing the expandable member 670. For example, the proximal end portion of the tubular member 671 can be coupled to a source of an inflation medium.

The expandable member 670 has a collapsed configuration (illustrated in FIG. 15) and an expanded configuration (illustrated in FIG. 14). When in its collapsed configuration, the expandable member 670 contains substantially no inflation medium and can allow the head 620 and expandable member 670 to be easily inserted and maneuvered within a patient's body. Once at a desired location, the expandable member 670 can be moved to its expanded configuration by inflating the expandable member 670 with an inflation medium. In its expanded configuration, the expandable member 670 can maintain a position of the head 620 at a desired treatment site within the patient's body while the suturing device 600 is actuated to place a needle and suture through a targeted tissue.

The expandable member 670 can be inflated or deflated by, for example an electric pump (not shown), however, any other suitable inflation or deflation means can be used. Similarly, any suitable external pressure regulating means may be used to regulate the pressure of the expandable member 670.

Although the guide wire 690 is illustrated in FIGS. 14 and 15 as extending through a retaining portion similar to the retaining portion 460 (e.g., through lumens defined by the head and elongate flexible sheath), it should be understood that other configurations are possible. For example, the retaining portion can include one or more retaining members (e.g., 566) disposed on an exterior surface of the head 620 and/or elongate flexible sheath 630, as shown in FIG. 11.

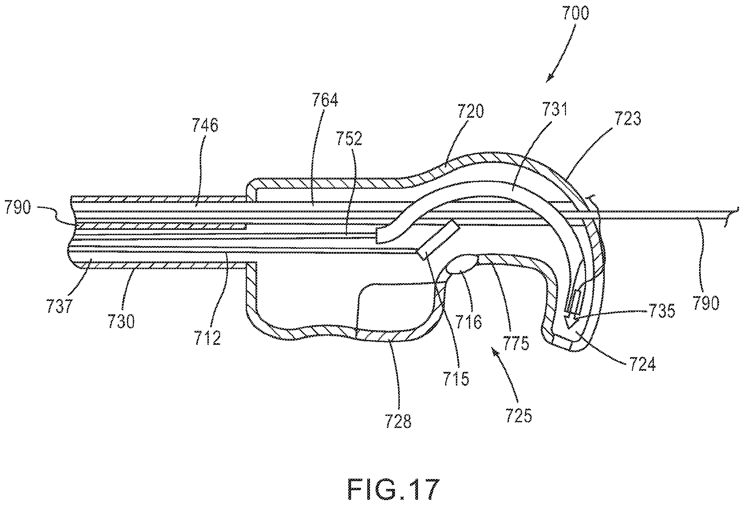

In some examples, a suturing device can include an imaging device such that the suturing device can be used for suturing tissue and for imaging an interior region of a patient's body. As illustrated in FIGS. 16 and 17, a lens assembly 716 and an imager 715 are coupled to a head 720 of a suturing device 700, and are configured to send image signals associated with an image of the interior of the patient's body. The suturing device 700 can also be coupled to a control device (not shown) used to control the operation of the imager 715. For example, the control device can include an image controller for operating the imager 715, and a processor for processing image signals transmitted from the imager 715. The control device can also include a monitor or be coupled to a monitor (not shown) for viewing the processed images. In alternative embodiments, other types of imaging devices can be incorporated. For example, an imaging device using optical fibers can be used.

As with previous embodiments, the head 720 is coupled to an elongate flexible sheath 730 and an actuator that includes an actuation cable 752 disposed within a lumen 737 of the flexible elongate sheath 730, as shown in FIG. 17. The suturing device 700 also includes a carrier member 731 and a catch member 728. The actuation of the suturing device 700 is functionally and structurally similar to the previous embodiments and thus will not be described in detail below. Also shown in FIG. 17, in this example the elongate flexible sheath 730 defines a lumen 746, and the head 720 defines a lumen 764 collectively configured to receive a guide wire 790, similar to the embodiment illustrated in FIG. 11.

As shown in FIG. 16 and 17, the lens assembly 716 is disposed on a wall 775 of the head 720 within an opening 725 defined in part by a curved portion 723 of the head 720. The lens assembly 716 defines a focal plane (not shown). The imager 715 is coupled to the head 720 such that the imager 715 is aligned within the focal plane associated with the lens assembly 716. The imager 715 can be coupled to the head 720 by any suitable coupler, for example, a rubber mount, an adhesive, a combination thereof, etc.

As shown in FIG. 17, an elongate tubular member 712 extends proximally from the imager 715 within the lumen 737 of the elongate flexible sheath 730. The tubular member 712 defines a lumen therethrough (not shown) that is configured to receive power cords, signal cords, or the like for transferring signals to and from the imager 715. In some embodiments, the tubular member 712 is configured to operatively connect the imager 715 to a visual display, power source, etc. (not shown) or a control device (not shown) as described above.

The imager 715 is configured to produce an image focused by the lens assembly 716 to be output to a user. The location of the lens assembly 716 disposed within the opening 725 enables a user to view the targeted tissue location for which suturing will be performed. For example, the lens assembly 716 can aid in positioning the opening 725 of the head 720 at a desired location to pass a needle 735 (shown loaded within a channel 724 of the head 720). The imager 715 can be a variety of different types of imaging device, including for example, a charge coupled device (CCD), a complementary metal-oxide-semiconductor (CMOS) sensor, an active pixel sensor, a thermal imaging sensor, a video camera tube, a gamma camera sensor, an x-ray sensor, or the like.

Although in this example, the elongate flexible sheath 730 is illustrated as defining two lumens (lumen 746 and lumen 737), it should be understood that the elongate flexible sheath 730 can define any number of lumens, such as, for example, one lumen or more than two lumens. For example, in some examples, the tubular member 712 can extend through a different lumen than the actuation cable 752.

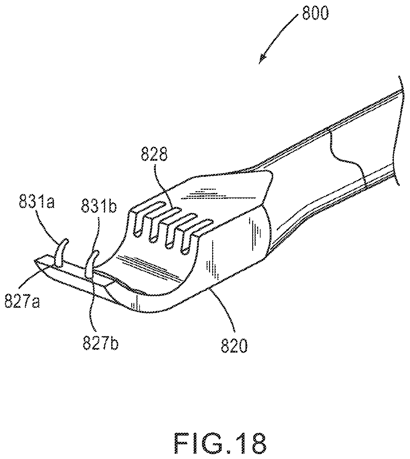

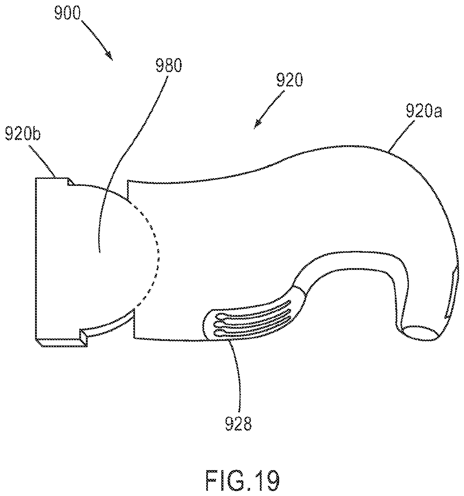

In some embodiments, a suturing device can include multiple carrier members configured to retain and pass multiple needles through tissue. As illustrated in FIG. 18, a suturing device 800 includes a first carrier member 831a movably disposed within a distal end portion 822 of a head 820. The first carrier member 831a is configured to frictionally retain a first needle (not shown). A second carrier member 831b is also movably disposed within the distal end portion 822 of the head 820. The second carrier member 830b is configured to frictionally retain a second needle (not shown). The distal end portion 822 defines first and second needle exit ports, 827a and 827b, respectively. A proximal portion of the first carrier member 831a and a proximal portion of the second carrier member 831b are coupled to an actuation cable (not shown), and the distal portions of carrier members 831a and 831b are configured to be moved or extended out of the needle exit port 827a and 827b, as shown in FIG. 18, when the suturing device is actuated. The carrier members 831a and 83 Ib, can be actuated in a similar manner as described above for other embodiments to pass a needle and suture through a desired tissue site and into a catch member 828. Other suturing devices configured to pass multiple needles through tissue that can be included on a suturing device as described herein are described in U.S. Patent Nos. 7,232,447, 6,995,643, and 7,041,111, and U.S. Patent Pub. No. 2006/0195121. The suturing device 800 can also be configured to be removably coupled to an elongate medical device, such as an endoscope, as described above.
In some examples, referring, for example, to FIG. 19, a suturing device can include an articulation mechanism to further facilitate positioning of the head of the suturing device within a patient's body. As illustrated in FIG. 19, a suturing device 900 includes a head 920 that includes a first portion 920a and a second portion 920b. The second portion 920b is configured to be coupled to a flexible elongate sheath (not shown). The suturing device 900 can also be removably coupled to an elongate medical device as described above (e.g., endoscope, sheath) (not shown in FIG. 19). The head 920 further includes an articulation mechanism 980 disposed between the first portion 920a of the head 920 and the second portion 920b of the head 920. The articulation mechanism 980 is configured to allow the first portion 920a to move relative the second portion 920b. For example, the first portion 920a can rotate relative to the second portion 920b about a pivot joint (within articulation mechanism) between the first portion 920a and the second portion 920b. The actuation mechanism 980 can further facilitate access to deep and/or difficult to reach areas within the patient. The suturing device 900 also includes a carrier member (not shown), a catch 928 and an actuator (not shown) and can be actuated to place sutures within tissue as described above for previous embodiments. Other examples of a head that can articulate or pivot that can be included in a suturing device as described herein are described in U.S. Patent Pub. No. 2004/0181243.

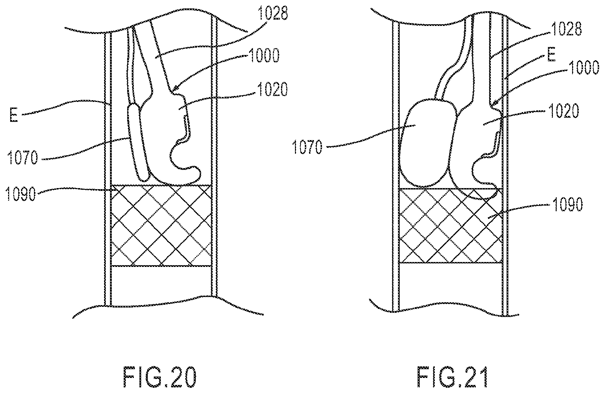

FIGS. 20 and 21 illustrate an example use of a suturing device as described herein. As shown in FIG. 20, a suturing device 1000 includes a head 1020, a flexible elongate sheath 1028, an expandable member 1070 and an actuator (not shown). In this example, the suturing device 1000 is used to secure an esophageal stent 1090 within an esophagus E (shown schematically). As shown in FIG. 20, the suturing device 1000 is inserted through the esophagus E with the expandable member 1070 in a collapsed configuration. As shown in FIG. 21, the suturing device 1000 can be positioned at a desired location, such as partially within an interior of the esophageal stent 1090 and adjacent a wall of the esophagus E. With the suturing device 1000 in a desired position, the expandable member 1070 can then be moved to the expanded configuration as described above (e.g., by inflating the expandable member 1070). In the expanded configuration, the expandable member 1070 can be used to maintain a position of the suturing device 1000 within the esophagus E. The suturing device 1000 can then be actuated (as described above in more detail relative to the operation of the suturing device 200) to place a suture through a portion of the stent 1090 and a portion of tissue along the wall of the esophagus E. Multiple sutures can be placed as needed by repeatedly actuating the suturing device 1000. For example, the head 1020 of the suturing device 1000 can be repositioned to a different portion of the stent 1090 and then actuated again to place a suture. In some embodiments as described above, a suturing device can be configured to place multiple sutures at one time.

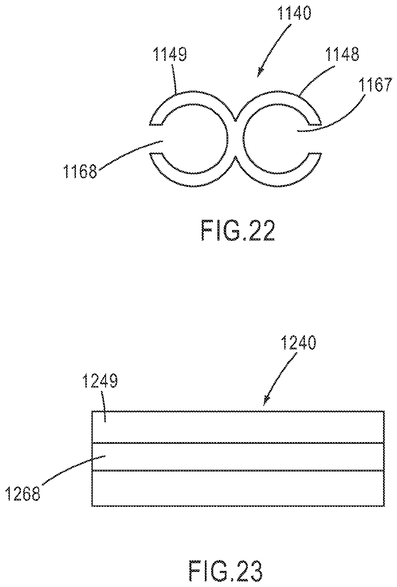

FIGS. 22 and 23 each illustrate an example of a coupling member that can be used to removably couple a suturing device to another medical device as described herein. Fig. 22 is an end view of a coupling member 1140 that includes a first clamp portion 1148 that defines an interior region 1167 configured to receive a portion of a suturing device (not shown), and a second clamp portion 1149 configured to receive a portion of an elongate medical device (e.g., an endoscope) (not shown). The coupling member 1140 can be a variety of different lengths. One or more coupling members 1140 can be used to removably couple a suturing device to an elongate medical device.

FIG. 23 is a side view of a coupling member 1240 that has a similar cross-section as the coupling member 1140. In this embodiment, the coupling member 1240 includes an elongate body that includes a first clamp portion 1249 that defines an interior region configured to receive a portion of a suturing device (not shown) and a second clamp portion (not shown) on an opposite side configured to receive a portion of an elongate medical device (e.g., an endoscope) (not shown). The coupling member 1240 can have a length such that the coupling member 1240 can extend along an entire length of a suturing device and/or the elongate medical device, or to extend along only a portion of a length of the suturing device and/or the elongate medical device. The clamp portions of both the coupling member 1140 and the coupling member 1240 can each have a different length. For example, the coupling portion 1148 can be a first length and the coupling portion 1149 can be a different length.

The coupling members 1140 and 1240 are merely examples of coupling members that can be used to couple a suturing device to another medical device. As described previously, other types of coupling methods can be used (e.g., band(s)). In some embodiments, a coupling member can include one or more closed sheath portions (not shown) rather than the open clamp portions as shown in FIG. 22. In such an embodiment, a suturing device or another elongate medical device can be slidably received through the sheath portion. The sheath portions can also be a variety of different lengths.

The various embodiments and examples of a suturing device described herein (e.g., 100, 200, 300, 400,. etc.) can be constructed with any suitable material used for such medical devices. For example, the various components of a suturing device can be formed with one or more biocompatible materials, such as silicone, nylon, polyglycolic acid, or stainless steel, and various polymers. The various components of a suturing device can be formed with various elastic materials, flexible materials, rubber materials, or combinations thereof. In some embodiments, the elongate sheath (e.g., 130, 230, 330, etc.) can be formed with cuts or scoring along its length and/or width to provide for flexibility of the sheath.

In addition, various components of a suturing device can be fabricated from extruded, molded, or machined plastic material(s), such as polypropylene, polycarbonate, or glass-filled polycarbonate. Some components may be made of stainless steel. For example, the catch member (e.g., 228, 328, 428, etc.) can be constructed, with thin stainless steel of high temper, such as ANSI 301 full hard. The catch member can be fabricated, for example, by way of stamping, laser machining, or chemical etching. Other suitable materials will be apparent to those skilled in the art.

The material(s) used to form the suture used with a suturing device described herein should be biocompatible. In some embodiments, the sutures can be made with, for example, a biodegradable material. For example, in some medical procedures, it maybe desirable to place sutures that over time can biodegrade within the patient's body. In another example, in some situations where sutures are used to secure an implant or stent as described herein, it may be desirable for the suture to biodegrade to enable easier removal of the implant or stent after a specified time period. Various types of suture material commonly used in medical procedures can be used with a suturing device as described herein.
While various embodiments of the invention have been described above, it should be understood that they have been presented by way of example only, and not limitation. Thus, the breadth and scope of the invention should not be limited by any of the above- described embodiments, but should be defined only in accordance with the following claims and their equivalents.
The previous description of the various embodiments is provided to enable a person skilled in the art to make and/or use the invention. While certain embodiments of the invention have been particularly shown and described, these disclosed embodiments are illustrative of and not limiting on the invention.
For example, the suturing devices described herein (e.g., 100, 200, 300, etc.) can include various combinations and/or sub-combinations of any of the components and/or features of the different embodiments described herein. For example, any of the embodiments of a suturing device can include a retaining portion for receiving a guide wire as described herein, and/or also a coupling member (e.g., 140, 240, etc.) to removably couple the suturing device to an elongate medical device, such as an endoscope. In another example, any of the embodiments of a head can be configured to include an imaging device or an articulation mechanism. An expandable member can also be included in any of the embodiments of a suturing device.
Although various configurations of a head were illustrated and described, other configurations of a head and suturing mechanism (e.g., carrier member and catch member) can be configured to be coupled to an elongate flexible sheath (e.g., 130, 230, 330. etc.) and an actuator (e.g., 150, 250, 350) as described herein. For example, various different types of heads and suturing mechanisms are described in U.S. Patent Nos. 5,741,277, 7,122,039, 6,346,111, 7,060,077, 7,033,370, 7,232,447, and 6,936,054, and in U.S. patent Publication No. 2006/0206119. Such heads and suturing mechanisms can be incorporated into a suturing device as described herein for use, for example, in endoscopic applications.

The suturing devices described herein can be used to suture various types of tissue in various locations within a patient's body. For example, the suturing devices can be used in a body lumen. The suturing devices can also be used to suture and/or secure various types of stents and implants within a patient's body, including for example, implants for pelvic floor applications and stents such as, for example, TB, duodenal, biliary or colonic stents.

## Claims

1. A suturing device (200), comprising:
a head (220) including a proximal end portion (221) and a distal end portion (222), the distal end portion (222) defining a first opening (227), the proximal end portion (221) defining a second opening (226);
a curved carrier member (231) movably disposed at least partially within the distal end portion (222) of the head (220), the carrier member (231) being movable from a first position in which the carrier member (231) is disposed substantially within the distal end portion (222) of the head (220) to a second position in which a first portion of the carrier member (231) is moved out through the first opening (227) and disposed adjacent the second opening (226) and a second portion of the carrier member (231) is disposed within the distal end portion (222) of the head (220), the carrier member (231) being configured to receive a needle (235) coupled to a suture (236) when in the first position and configured to pass the needle (235) and a portion of the suture (236) through a tissue when moved from the first position to the second position; **characterized in that** the proximal end portion (221) of the head (220) is configured to be removably coupled to a distal end portion of an elongate medical device (210) configured to be disposed within a body lumen of a patient; and **in that** the suturing device further comprises
an elongate flexible sheath (230) coupled to the proximal end portion (221) of the head (220); and
a coupling member (240) configured to removably couple the proximal end portion (221) of the head (220) to the distal end portion of the elongate medical device (210),;
wherein when the proximal end portion (221) of the head (220) is coupled to the distal end portion of the elongate medical device (210), the elongate flexible sheath (230) extends along an exterior of the elongate medical device (210).

2. The suturing device of claim 1, wherein the carrier member (231) defines an opening configured to receive a needle (235) coupled to a suture (236).

3. The suturing device of claim 1, further comprising: an actuation cable (252) coupled to the carrier member (231), the actuation cable being disposed at least partially within a lumen (237) defined by the elongate flexible sheath (230).

4. The suturing device of claim 1, further comprising: an actuation cable (252) coupled to the carrier member (231) and configured to extend through a lumen (237) of the medical device (210) when the head (220) is removably coupled thereto.

5. The suturing device of claim 1, wherein the carrier member (231) is a first carrier member, the medical device (210) further comprising: a second carrier member movably disposed at least partially within the distal end portion (222) of the head (220).

6. The suturing device of claim 1 , wherein the carrier member (231) when moved between its first position and its second position is configured to rotate about an axis transverse to a longitudinal axis defined by the head (220).

7. The suturing device of claim 1, wherein the head (220) includes a first portion and a second portion, the first portion configured to move relative to the second portion.

## Patentansprüche

1. Nähvorrichtung (200), mit:
einem Kopf (220), der einen proximalen Endabschnitt (221) und einen distalen Endabschnitt (222) aufweist, wobei der distale Endabschnitt (222) eine erste Öffnung (227) definiert und der proximale Endabschnitt (221) eine zweite Öffnung (226) definiert;
einem gekrümmten Trägerelement (231), das mindestens teilweise innerhalb des distalen Endabschnitts (222) des Kopfes (220) beweglich angeordnet ist, wobei das Trägerelement (231) von einer ersten Position, in der das Trägerelement (231) im Wesentlichen innerhalb des distalen Endabschnitts (222) des Kopfes (220) angeordnet ist, zu einer zweiten Position beweglich ist, in der ein erster Abschnitt des Trägerelements (231) durch die erste Öffnung (227) heraus bewegt ist und benachbart zur zweiten Öffnung (226) angeordnet ist und ein zweiter Abschnitt des Trägerelements (231) innerhalb des distalen Endabschnitts (222) des Kopfes (220) angeordnet ist, wobei das Trägerelement (231) konfiguriert ist, eine Nadel (235) aufzunehmen, die mit einem Nahtmaterial (236) gekoppelt ist, wenn es sich in der ersten Position befindet, und konfiguriert ist, die Nadel (235) und einen Abschnitt des Nahtmaterials (236) durch eine Gewebe zu führen, wenn es von der ersten Position zur zweiten Position bewegt wird;
**dadurch gekennzeichnet, dass** der proximale Endabschnitt (221) des Kopfes (220) konfiguriert ist, entfernbar mit einem distalen Endabschnitt einer länglichen medizinischen Vorrichtung (210) gekoppelt zu werden, die konfiguriert ist, innerhalb eines Körperlumens eines Patienten angeordnet zu werden; und
dass die Nähvorrichtung ferner eine längliche flexible Hülle (230), die mit dem proximalen Endabschnitt (221) des Kopfes (220) gekoppelt ist; und
ein Kopplungselement (240) aufweist, das konfiguriert ist, den proximalen Endabschnitt (221) des Kopfes (220) entfernbar mit dem distalen Endabschnitt der länglichen medizinischen Vorrichtung (210) zu koppeln;
wobei, wenn der proximale Endabschnitt (221) des Kopfes (220) mit dem distalen Endabschnitt der länglichen medizinischen Vorrichtung (210) gekoppelt ist, sich die längliche flexible Hülle (230) längs eines Äußeren der länglichen medizinischen Vorrichtung (210) erstreckt.

2. Nähvorrichtung nach Anspruch 1, wobei das Trägerelement (231) eine Öffnung definiert, die konfiguriert ist, eine Nadel (235) aufzunehmen, die mit einem Nahtmaterial (236) gekoppelt ist.

3. Nähvorrichtung nach Anspruch 1, die ferner aufweist: ein Betätigungskabel (252), das mit dem Trägerelement (231) gekoppelt ist, wobei das Betätigungskabel mindestens teilweise innerhalb eines Lumens (237) angeordnet ist, das durch die längliche flexible Hülle (230) definiert wird.

4. Nähvorrichtung nach Anspruch 1, die ferner aufweist: ein Betätigungskabel (252), das mit dem Trägerelement (231) gekoppelt und konfiguriert ist, sich durch ein Lumen (237) der medizinischen Vorrichtung (210) zu erstrecken, wenn der Kopf (220) entfernbar damit gekoppelt ist.

5. Nähvorrichtung nach Anspruch 1, wobei das Trägerelement (231) ein erstes Trägerelement ist, wobei die medizinische Vorrichtung (210) ferner aufweist: ein zweites Trägerelement, das mindestens teilweise innerhalb des distalen Endabschnitts (222) des Kopfes (220) beweglich angeordnet ist.

6. Nähvorrichtung nach Anspruch 1, wobei das Trägerelement (231) konfiguriert ist, wenn es zwischen seiner ersten Position und seiner zweiten Position bewegt wird, sich um eine Achse zu drehen, die transversal zu einer Längsachse ist, die durch den Kopf (220) definiert wird.

7. Nähvorrichtung nach Anspruch 1, wobei der Kopf (220) einen ersten Abschnitt und einen zweiten Abschnitt aufweist, wobei der erste Abschnitt konfiguriert ist, sich relativ zum zweiten Abschnitt zu bewegen.

## Revendications

1. Dispositif de suture (200), comprenant :
une tête (220) présentant une partie d'extrémité proximale (221) et une partie d'extrémité distale (222), la partie d'extrémité distale (222) définissant une première ouverture (227), la partie d'extrémité proximale (221) définissant une deuxième ouverture (226) ;
un élément porteur incurvé (231) disposé de manière mobile au moins en partie à l'intérieur de la partie d'extrémité distale (222) de la tête (220), ledit élément porteur (231) étant déplaçable d'une première position où l'élément porteur (231) est disposé sensiblement à l'intérieur de la partie d'extrémité distale (222) de la tête (220) vers une deuxième position où une première partie de l'élément porteur (231) est sortie par la première ouverture (227) et adjacente à la deuxième ouverture (226), et une deuxième partie de l'élément porteur (231) est disposée à l'intérieur de la partie d'extrémité distale (222) de la tête (220), l'élément porteur (231) étant prévu pour recevoir une aiguille (235) reliée à un fil de suture (236) quand il est en première position, et prévu pour que l'aiguille (235) et une partie du fil de suture (236) traversent un tissu quand il est déplacé de la première position vers la deuxième position ;
**caractérisé en ce que** la partie d'extrémité proximale (221) de la tête (220) est prévue pour être raccordée de manière amovible à une partie d'extrémité distale d'un dispositif médical longiforme (210) destiné à être mis en place à l'intérieur d'une lumière corporelle d'un patient ; et **en ce que** le dispositif de suture comprend en outre une gaine longiforme flexible (230) raccordée à la partie d'extrémité proximale (221) de la tête (220) ; et
un élément d'accouplement (240) prévu pour raccorder de manière amovible la partie d'extrémité proximale (221) de la tête (220) à la partie d'extrémité distale du dispositif médical longiforme (210) ;
où, quand la partie d'extrémité proximale (221) de la tête (220) est raccordée à la partie d'extrémité distale du dispositif médical longiforme (210), la gaine longiforme flexible (230) s'étend extérieurement le long du dispositif médical longiforme (210).

2. Dispositif de suture selon la revendication 1, où l'élément porteur (231) définit une ouverture prévue pour recevoir une aiguille (235) reliée à un fil de suture (236).

3. Dispositif de suture selon la revendication 1, comprenant en outre : un câble d'actionnement (252) relié à l'élément porteur (231), ledit câble d'actionnement étant disposé au moins en partie à l'intérieur d'une lumière (237) définie par la gaine longiforme flexible (230).

4. Dispositif de suture selon la revendication 1, comprenant en outre : un câble d'actionnement (252) relié à l'élément porteur (231) et prévu pour s'étendre dans une lumière (237) du dispositif médical (210) quand la tête (220) est raccordée de manière amovible à celui-ci.

5. Dispositif de suture selon la revendication 1, où l'élément porteur (231) est un premier élément porteur, le dispositif médical (210) comprenant en outre : un deuxième élément porteur disposé de manière mobile au moins en partie à l'intérieur de la partie d'extrémité distale (222) de la tête (220).

6. Dispositif de suture selon la revendication 1, où, quand il est déplacé entre sa première position et sa deuxième position, l'élément porteur (231) est prévu pour être rotatif autour d'un axe transversal à un axe longitudinal défini par la tête (220).

7. Dispositif de suture selon la revendication 1, où la tête (220) comprend une première partie et une deuxième partie, la première partie étant prévue pour être mobile par rapport à la deuxième partie.
